(19) Europäisches Patentamt / European Patent Office / Office européen des brevets

(11) **EP 2 834 761 B1**

(12) **FASCICULE DE BREVET EUROPEEN**

(45) Date de publication et mention de la délivrance du brevet:
**25.05.2016 Bulletin 2016/21**

(21) Numéro de dépôt: **13719985.7**

(22) Date de dépôt: **04.04.2013**

(51) Int Cl.:
*G06F 19/00* *(2011.01)*    *G06F 17/50* *(2006.01)*

(86) Numéro de dépôt international:
**PCT/FR2013/050746**

(87) Numéro de publication internationale:
**WO 2013/150247 (10.10.2013 Gazette 2013/41)**

(54) **PROCEDE DE SIMULATION D'AUTO ASSEMBLAGE DE COPOLYMERES A BLOCS POUR LA CONCEPTION D'UN CIRCUIT IMPRIME, PROCEDE DE CONCEPTION, SYSTEME DE CONCEPTION ET PROGRAMME D'ORDINATEUR CORRESPONDANTS**

VERFAHREN ZUR SIMULATION DER SELBSTANORDNUNG VON BLOCKCOPOLYMEREN ZUM ENTWURF EINER LEITERPLATTE, ENTSPRECHENDES ENTWURFSVERFAHREN, ENTWURFSSYSTEM UND COMPUTERPROGRAMM

METHOD FOR SIMULATING THE SELF-ASSEMBLY OF BLOCK COPOLYMERS IN ORDER TO DESIGN A PRINTED CIRCUIT, CORRESPONDING DESIGN METHOD, DESIGN SYSTEM AND COMPUTER PROGRAM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **05.04.2012 FR 1253161**

(43) Date de publication de la demande:
**11.02.2015 Bulletin 2015/07**

(73) Titulaire: **Commissariat à l'Énergie Atomique et aux Énergies Alternatives 75015 Paris (FR)**

(72) Inventeur: **BELLEDENT, Jérôme F-38240 Meylan (FR)**

(74) Mandataire: **Bonnet, Michel Cabinet Bonnet 93, rue Réaumur - Boîte 10 75002 Paris (FR)**

(56) Documents cités:
- **JOEL K. W. YANG ET AL: "Complex self-assembled patterns using sparse commensurate templates with locally varying motifs", NATURE NANOTECHNOLOGY, vol. 5, no. 4, 1 avril 2010 (2010-04-01), pages 256-260, XP055057907, ISSN: 1748-3387, DOI: 10.1038/nnano.2010.30**
- **MARK P. STOYKOVICH ET AL: "Directed Self-Assembly of Block Copolymers for Nanolithography: Fabrication of Isolated Features and Essential Integrated Circuit Geometries", ACS NANO, vol. 1, no. 3, 1 octobre 2007 (2007-10-01), pages 168-175, XP055057909, ISSN: 1936-0851, DOI: 10.1021/nn700164p**
- **KOSTAS CH. DAOULAS ET AL: "Directed assembly of copolymer materials on patterned substrates: Balance of simple symmetries in complex structures", JOURNAL OF POLYMER SCIENCE PART B: POLYMER PHYSICS, vol. 44, no. 18, 15 septembre 2006 (2006-09-15), pages 2589-2604, XP055057964, ISSN: 0887-6266, DOI: 10.1002/polb.20904**

# EP 2 834 761 B1

**Description**

La présente invention concerne un procédé de simulation d'un placement de motifs par auto assemblage de copolymères à blocs dans un contour imprimé sur plaque par lithographie. Elle concerne également un procédé de conception d'un circuit imprimé mettant en oeuvre ce procédé de simulation. Enfin, elle concerne également un système de conception et un programme d'ordinateur correspondants.

Le placement de motifs, tels que des lignes conductrices, des contacts ou des piliers, par auto assemblage de copolymères à blocs dans un contour préalablement imprimé par une technique de lithographie est une technique récente de conception de circuits imprimés électroniques, par exemple décrite dans l'article de Sanders et al, intitulé « Integration of directed self-assembly with 193 nm lithography », Journal of Photopolymer Science and Technology, vol. 23, N° 1 (2010), pages 11-18.

Cette technique consiste dans un premier temps à imprimer un premier motif sur plaque par une technique de lithographie connue, par exemple une photolithographie, une lithographie par faisceau d'électrons ou autre, le premier motif obtenu incluant au moins un contour imprimé dans lequel des motifs plus fins, par exemple de dimensions nanométriques, sont destinés à être placés. A cette échelle nanométrique et dans un deuxième temps, le placement des motifs plus fins se fait par auto assemblage de copolymères à blocs introduits dans le contour. Les motifs obtenus dépendent des ratios moléculaires choisis dans les copolymères et de la portion moléculaire supprimée après le placement effectué. Ainsi, à l'aide de copolymères à deux blocs A et B de natures différentes et reliés entre eux par une liaison covalente, il est possible selon les ratios choisis d'obtenir des lignes, des contacts, des piliers, des tranchées ou autres.

Cette technique permet, de cette façon, d'atteindre des résolutions nanométriques ouvrant des perspectives de miniaturisation des composants en microélectronique.

Mais cette technique doit être scrupuleusement mise en oeuvre pour contrôler spatialement avec précision le positionnement des motifs, d'une part pour répondre aux besoins du design, d'autre part pour respecter un alignement entre différents niveaux de circuit. Or, que ce soit au niveau des contours ou au niveau des motifs plus fins à l'intérieur des contours, les motifs sur plaques subissent les distorsions classiques induites par un manque de résolution, des variations de doses, de focus, des effets dans la résine, etc.

C'est la raison pour laquelle il est avantageux de pouvoir procéder à une simulation de placements de motifs par auto assemblage de copolymères à blocs dans des contours imprimés sur plaque par lithographie, en complément d'une correction optique de proximité dite OPC (de l'anglais « Optical Proximity Correction »). Cela permet en effet d'anticiper les distorsions (objectif principal d'une OPC), puis d'affiner cette anticipation par une simulation des placements de motifs.

Il existe notamment des propositions de simulation des effets physico-chimiques entrant en jeu dans l'auto assemblage de copolymères à blocs. De la sorte, il peut être envisagé de simuler le placement de motifs sur la base d'une telle simulation d'effets physico-chimiques. Mais cette approche est très complexe et coûteuse en temps de calcul. Ainsi elle ne peut être appliquée qu'à de très petits volumes de copolymères.

Il peut ainsi être souhaité de prévoir un procédé de simulation du type précité qui permette de s'affranchir d'au moins une partie des problèmes et contraintes précités.

Il est donc proposé un procédé de simulation d'un placement de motifs par auto assemblage de copolymères à blocs dans un contour imprimé sur plaque par lithographie, comportant les étapes suivantes :

- extraction de paramètres géométriques du contour enregistrés en mémoire,
- sélection, par un processeur ayant accès à la mémoire, d'au moins un extremum local d'une figure d'interférence produite à l'intérieur du contour à partir des paramètres géométriques du contour par application d'un modèle de propagation d'ondes interférant entre elles,
- fourniture, par le processeur et à partir de cet extremum local, de paramètres de placement d'au moins un motif destiné à être obtenu par auto assemblage de copolymères à blocs dans le contour.

Il a en effet été constaté, de façon surprenante, qu'un modèle de propagation n'ayant a priori pas de corrélation avec les phénomènes physico-chimiques mis en jeu dans le placement de motifs par auto assemblage de copolymères à blocs dans des contours donnés, est finalement particulièrement adapté pour anticiper le placement de ces motifs, ceux-ci étant localisables à partir des extrema locaux de figures d'interférences produites par ce modèle de propagation. Comme les interférences entre ondes se propageant selon un modèle donné sont en outre simples à calculer en tous points, il en résulte une estimation du placement de motifs par auto assemblage de copolymères à blocs qui est à la fois précise et simple à calculer.

De façon optionnelle :

- les paramètres géométriques du contour sont des points de ce contour,

- l'étape de sélection comporte l'application d'un premier modèle de propagation d'ondes émises à partir de ces points, produisant une figure d'interférence à l'intérieur du contour, et la sélection d'au moins un maximum local de la partie imaginaire de cette figure d'interférence.

De façon optionnelle également, la figure d'interférence produite par le premier modèle, notée $I_0(M)$ en un point M quelconque situé à l'intérieur du contour, est définie par l'expression suivante :

$$I_0(M) = \sum_{i=0}^{n-1} f\left(\left\|\overrightarrow{p_i M}\right\|\right) e^{\frac{2i\pi\left(\left\|\overrightarrow{p_i M}\right\| - d_1\right)}{pn_0}},$$

où f est une fonction d'atténuation définie de la façon suivante :

$$f(r) = \begin{cases} 1 & si \quad r \le d_1 \\ e^{-\frac{(r-d_1)^2}{2d_2^2}} & si \quad r > d_1 \end{cases},$$

et où $\{p_i, 0 \le i < n\}$ est un ensemble de points du contour formant ses paramètres géométriques, le paramètre $d_1$ est un paramètre constant, par exemple choisi proche de la moitié d'un pas naturel desdits copolymères à blocs, ce pas naturel représentant une distance naturelle entre copolymères à blocs à l'équilibre énergétique, et le paramètre $d_2$ est un paramètre constant, par exemple choisi proche ce pas naturel.

De façon optionnelle également, les paramètres $d_1$ et $d_2$ sont définis à partir d'une calibration dudit modèle de propagation sur quelques motifs

De façon optionnelle également, l'étape de sélection comporte en outre, suite à l'application dudit premier modèle de propagation et à la sélection d'au moins un maximum local de la partie imaginaire de la figure d'interférence produite par ce premier modèle, une boucle d'étapes comportant l'application d'un nouveau modèle de propagation d'ondes, chaque itération de cette boucle d'étapes comportant :

- l'application du nouveau modèle par émissions d'ondes à partir d'au moins un maximum local sélectionné à l'itération précédente ou à partir dudit au moins un maximum local sélectionné par application du premier modèle s'il s'agit de la première itération, produisant une nouvelle figure d'interférence à l'intérieur du contour,
- la sélection d'au moins un maximum local de la partie imaginaire de cette nouvelle figure d'interférence.

De façon optionnelle également, la nouvelle figure d'interférence, notée $I_j(M)$ en un point M quelconque situé à l'intérieur du contour et à la j-ème itération de la boucle d'étapes, est définie par l'expression suivante :

$$I_j(M) = I_0(M) + C_0 \sum_{E \in E_{j-1}} g\left(\left\|\overrightarrow{EM}\right\|\right) e^{\frac{2i\pi\left(\left\|\overrightarrow{EM}\right\| - 0,75 pn_0\right)}{pn_0}},$$

où la fonction d'atténuation g est définie de la façon suivante :

$$g(r) = \begin{cases} 1 & si \quad r \le pn_0 \\ e^{-\frac{(r-pn_0)^2}{2d_2^2}} \cdot \left(1 - e^{-\frac{9r^2}{2pn_0^2}}\right) & si \quad r > pn_0 \end{cases},$$

et où $C_0$ est un paramètre constant, par exemple choisi proche de 1, et $E_{j-1}$ est l'ensemble des maxima locaux sélectionnés à l'itération précédente ou par application du premier modèle s'il s'agit de la première itération.

De façon optionnelle également, le paramètre $C_0$ est défini à partir d'une calibration dudit modèle de propagation sur quelques motifs

De façon optionnelle également, l'étape de sélection s'arrête lorsque le nombre d'extrema locaux sélectionnés atteint un nombre attendu de motifs à placer dans le contour par auto assemblage de copolymères à blocs, ce nombre attendu étant par exemple défini comme étant le ratio entre la surface intérieure du contour et un espace naturel moyen connu, occupé par chacun de ces motifs dans un environnement sans contrainte de contour, pour leur placement par auto assemblage de copolymères à blocs.

Il est également proposé un procédé de conception d'un circuit imprimé destiné à comporter au moins un contour imprimé sur plaque par lithographie, au moins un motif devant être placé par auto assemblage de copolymères à blocs dans ce contour, comportant les étapes suivantes :

- enregistrement en mémoire de paramètres géométriques d'une pluralité de contours de formes différentes,
- exécution des étapes d'un procédé de simulation tel que défini précédemment pour chacun desdits contours, et
- sélection d'un contour à partir des paramètres de placement fournis pour chacun desdits contours, sur la base d'un placement souhaité prédéfini d'au moins un motif dans le circuit imprimé.

De façon optionnelle, un procédé de conception d'un circuit imprimé selon l'invention peut en outre comporter les étapes suivantes :

- impression du contour sélectionné sur une plaque du circuit imprimé par photolithographie, et
- insertion et auto assemblage de copolymères à blocs dans ce contour imprimé.

Il est également proposé un programme d'ordinateur téléchargeable depuis un réseau de communication et/ou enregistré sur un support lisible par ordinateur et/ou exécutable par un processeur, caractérisé en ce qu'il comprend des instructions pour l'exécution des étapes d'un procédé de simulation ou pour l'exécution des étapes d'un procédé de conception tels que définis précédemment, lorsque ledit programme est exécuté sur un ordinateur.

Enfin, il est également proposé un système de conception de circuit imprimé, comportant :

- une mémoire de stockage de paramètres géométriques d'une pluralité de contours de formes différentes,
- un simulateur programmé pour la mise en oeuvre d'un procédé de simulation tel que défini précédemment pour chacun desdits contours, et
- un sélecteur de contour à partir des paramètres de placement fournis pour chacun desdits contours, sur la base d'un placement souhaité prédéfini d'au moins un motif dans le circuit imprimé.

L'invention sera mieux comprise à l'aide de la description qui va suivre, donnée uniquement à titre d'exemple et faite en se référant aux dessins annexés dans lesquels :

- la figure 1 représente schématiquement la structure générale d'un système de conception de circuit imprimé, selon un mode de réalisation de l'invention,
- la figure 2 illustre les étapes successives d'un procédé de simulation mis en oeuvre par le système de conception de la figure 1,
- la figure 3 illustre un positionnement naturel de copolymères à blocs pour un arrangement cylindrique vertical théorique sans contrainte de contour,
- la figure 4 illustre le résultat d'une exécution du procédé de simulation de la figure 2 sur un contour donné, et
- la figure 5 illustre les étapes successives d'un procédé de conception de circuit imprimé, selon un mode de réalisation de l'invention.

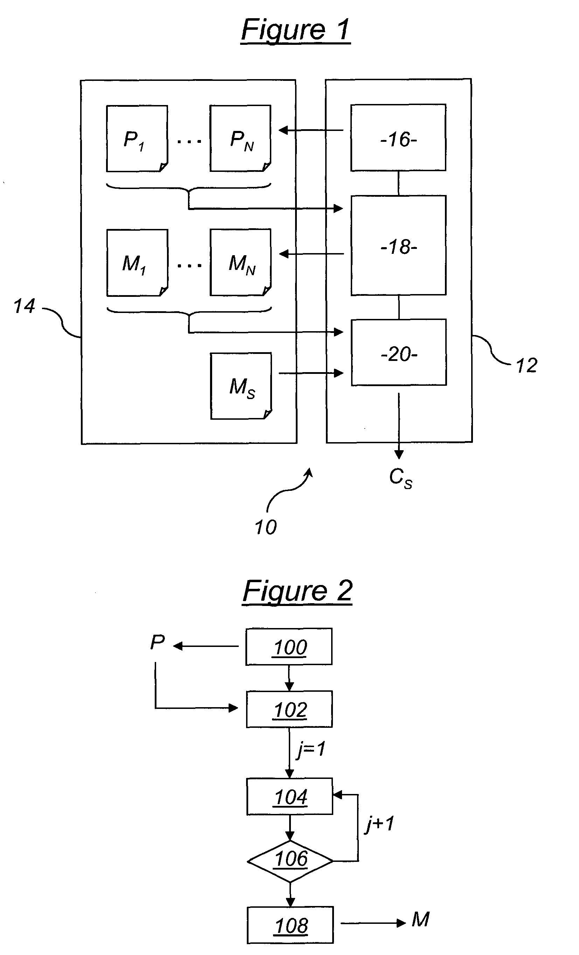

Le système 10 de conception de circuit imprimé représenté schématiquement sur la figure 1 comporte un module de traitement 12 associé de façon classique à une mémoire 14 (par exemple une mémoire RAM). Il peut par exemple être mis en oeuvre dans un dispositif informatique tel qu'un ordinateur classique comportant un processeur associé à une ou plusieurs mémoires pour le stockage de fichiers de données et de programmes d'ordinateurs. Le module de traitement 12 peut alors lui-même être considéré comme formé d'un processeur associé à une mémoire de stockage des instructions qu'il exécute sous forme de programmes d'ordinateurs.

Le module de traitement 12 tel qu'illustré sur la figure 1 comporte ainsi fonctionnellement trois programmes d'ordinateurs 16, 18 et 20.

Le premier programme d'ordinateur 16 est un programme de génération et d'enregistrement de paramètres géométriques de contours. A partir d'un contour C à imprimer sur plaque, par exemple défini et corrigé par OPC, il est conçu pour fournir un ensemble P de paramètres géométriques de ce contour C sous la forme d'un fichier numérique.

Dans le mode de réalisation qui va être détaillé, ces paramètres sont des points $p_i$ du contour C répartis le plus régulièrement possible le long de ce contour.

Le contour C lui-même peut être fourni sous forme d'image numérique issue d'une simulation, d'une observation par microscope électronique à balayage, etc.

L'ensemble $P = \{p_i, 0 \leq i < n\}$ est par exemple un ensemble de points appartenant à C tels que :

-

$$\forall i \in \{1, ..., n-1\}, D\left(\overrightarrow{p_{i-1}p_i}\right) = d_0 \text{ ,}$$

-

$$\forall i \in \{1, ..., n-2\}, D\left(\overrightarrow{p_{i-1}p_{i+1}}\right) > d_0 \text{ ,}$$

et

-

$$D\left(\overrightarrow{p_{n-1}p_1}\right) \leq d_0 \text{ ,}$$

où D désigne la distance curvilinéaire le long du contour C et $d_0$ est un paramètre positif dont la valeur réelle est proche du quart du pas naturel $pn_0$ séparant les microphases que génèrent les copolymères considérés sur une surface libre, le pas naturel $pn_0$ représentant la distance naturelle entre les copolymères à blocs considérés à l'équilibre énergétique.

La première condition impose une répartition régulière des points $p_i$ le long du contour C. La deuxième condition propose un sens de l'ensemble P des points $p_i$ le long du contour C. Enfin, la troisième condition définit les positions relatives des premier et dernier points de l'ensemble P dans le contour C qui est fermé.

Ainsi, à partir d'une pluralité de contours $C_i$, ..., $C_N$ de formes différentes, le premier programme d'ordinateur 16 fournit une pluralité d'ensembles $P_1$, ..., $P_N$ de paramètres géométriques respectifs de ces contours sous la forme de fichiers numériques. Ces fichiers numériques $P_1$, ..., $P_N$ sont alors stockés en mémoire 14.

Le deuxième programme d'ordinateur 18 est un simulateur programmé pour la mise en oeuvre d'un procédé de simulation qui sera détaillé en référence à la figure 2. A partir de chaque fichier $P_i$, il fournit un ensemble $M_i$ de paramètres de placement de motifs dans le contour $C_i$ considéré, ce placement de motifs correspondant, par l'efficacité de la simulation, à celui d'un auto assemblage de copolymères à blocs dans le contour $C_i$ considéré lorsque ce dernier est imprimé sur plaque par lithographie.

Ainsi, à partir de la pluralité d'ensembles $P_1$, ..., $P_N$ de paramètres géométriques, le deuxième programme d'ordinateur 18 fournit une pluralité d'ensembles $M_i$, .... $M_N$ de paramètres de placement de motifs respectifs dans les contours $C_1$, ..., $C_N$ sous la forme de fichiers numériques. Ces fichiers numériques $M_1$, ..., $M_N$ sont alors stockés en mémoire 14. Les paramètres de placement de motifs sont par exemple les coordonnées des centres des motifs placés par auto assemblage des copolymères à blocs.

Le troisième programme d'ordinateur 20 est un sélecteur de contour à partir de la pluralité d'ensembles $M_1$, ..., $M_N$ de paramètres de placement de motifs fournis pour chacun des contours $C_1$, ..., $C_N$, sur la base d'un placement souhaité prédéfini d'au moins un motif dans le circuit imprimé à concevoir. Ce placement souhaité est par exemple prédéfini sous la forme d'un fichier $M_S$ de paramètres définis de la même façon que ceux des fichiers $M_i$, .... $M_N$. Par une simple comparaison des paramètres du fichier $M_S$ avec les paramètres des fichiers $M_1$, ..., $M_N$, à l'aide d'un critère de distance quelconque par exemple, l'un de ces fichiers $M_1$, ..., $M_N$ est sélectionné et le contour correspondant aussi. Ce dernier est noté $C_S$ sur la figure 1.

On notera par ailleurs que les programmes d'ordinateurs 16, 18, 20 sont présentés comme distincts, mais cette distinction est purement fonctionnelle. Ils pourraient tout aussi bien être regroupés en un ou plusieurs logiciels. Leurs fonctions pourraient aussi être au moins en partie micro programmées ou micro câblées dans des circuits intégrés dédiés. Ainsi, en variante, le dispositif informatique mettant en oeuvre le système de conception 10 pourrait être remplacé par un dispositif électronique composé uniquement de circuits numériques (sans programme d'ordinateur) pour la réalisation des mêmes actions.

Le procédé de simulation illustré sur la figure 2 et mis en oeuvre par le simulateur 18 comporte une première étape 100 d'extraction d'un ensemble $P = \{p_i, 0 \leq i < n\}$ de paramètres géométriques d'un contour C enregistrés en mémoire 14. La référence P désigne ainsi l'un quelconque des fichiers $P_1$, ..., $P_N$ mentionnés précédemment.

Au cours d'une deuxième étape 102, le simulateur 18 du module de traitement 12 fournit une première figure d'interférence $I_0$ produite à l'intérieur A du contour C par la simulation d'une propagation d'ondes interférant entre elles à partir des points $p_i$ du contour C qui sont considérés comme des points d'émissions de ces ondes.

Un modèle de propagation fournissant de bons résultats est indiqué à titre d'exemple ci-dessous. Ce modèle est défini de telle sorte qu'en tout point M situé à l'intérieur A du contour fermé C, la première figure d'interférence $I_0$

prend la valeur suivante :

$$I_0(M) = \sum_{i=0}^{n-1} f\left(\left\|\overrightarrow{p_i M}\right\|\right) e^{\frac{2i\pi\left(\left\|\overrightarrow{p_i M}\right\| - d_1\right)}{pn_0}},$$

où la fonction d'atténuation f est définie de la façon suivante :

$$f(r) = \begin{cases} 1 & si \quad r \le d_1 \\ e^{-\frac{(r-d_1)^2}{2d_2{}^2}} & si \quad r > d_1 \end{cases}.$$

Dans ces deux expressions, le paramètre $d_1$ peut être choisi égal à la moitié du pas naturel $pn_0$ et le paramètre $d_2$ égal à $pn_0$. Il est possible également de calibrer ces paramètres à partir de résultats obtenus sur quelques structures élémentaires. On note ainsi que le modèle de propagation choisi impose une phase nulle en tout point de A situé à la distance $\dfrac{pn_0}{2}$ du contour C et une atténuation vers l'intérieur du contour C qui commence à cette même distance $\dfrac{pn_0}{2}$ du contour C.

Au cours de cette même étape 102, au moins un extremum local de la première figure d'interférence dans l'intérieur A du contour C, en l'occurrence un maximum local, est sélectionné. Soit $E_0$ l'ensemble des extrema locaux de $I_0$. Le critère de sélection, et donc de constitution de l'ensemble $E_0$, est par exemple le suivant :

$$E_0 = \left\{ M \in A \middle| \exists \varepsilon \in \mathfrak{R}^{+*} \middle| \forall M' \in A, \ 0 < \left\|\overrightarrow{MM'}\right\| < \varepsilon \Rightarrow \mathrm{Im}(I_0(M)) > \mathrm{Im}(I_0(M')) \right\},$$

où Im est la fonction donnant la partie imaginaire d'un nombre complexe quelconque. $E_0$ est donc l'ensemble des points de A pour lesquels un extremum local de la partie imaginaire de $I_0$ est atteint.

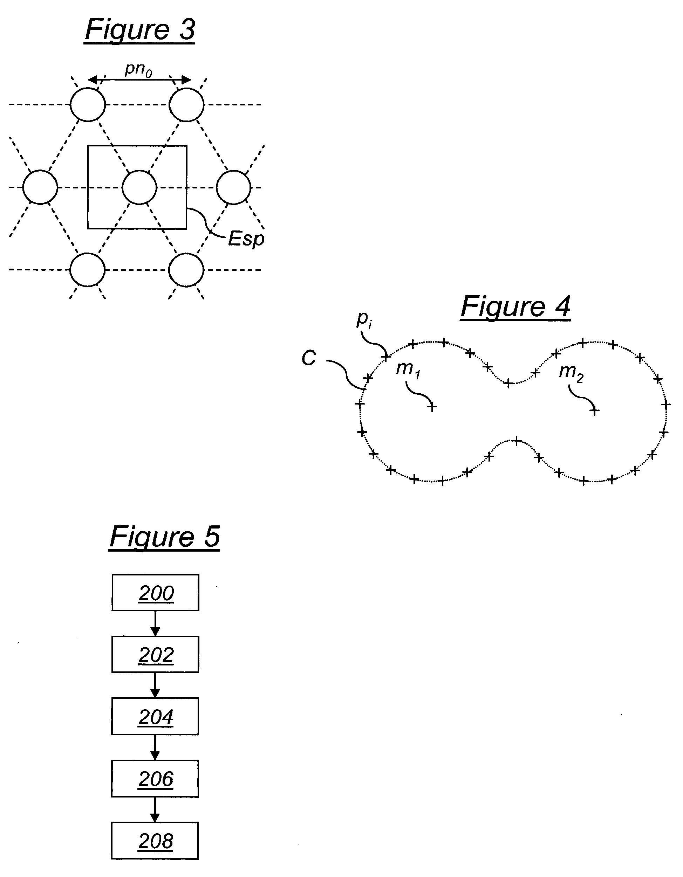

L'étape 102 est suivie d'une boucle d'étapes 104, 106 répétée autant de fois qu'un critère d'arrêt CA n'est pas satisfait. Le critère d'arrêt est par exemple le nombre attendu de motifs dans le contour C par auto assemblage de copolymères à blocs. Pour illustrer ce critère d'arrêt CA dans le cas particulier de motifs tels que des contacts ou piliers, un positionnement naturel de copolymères à blocs est représenté sur la figure 3 pour un arrangement cylindrique vertical théorique sans contrainte de contour. Selon cet arrangement, chaque contact ou pilier est distant des autres d'une distance égale au pas naturel $pn_0$, ce qui donne une structure régulière hexagonale dans laquelle chaque contact ou pilier occupe un espace naturel moyen Esp de $pn_0 \cdot \dfrac{\sqrt{3} pn_0}{2}$, soit $\dfrac{\sqrt{3} pn_0{}^2}{2}$. On peut supposer que l'espace occupé par chaque motif dans le contour C doit être aussi proche que possible de cet espace naturel moyen. Ainsi, le critère d'arrêt CA peut être donné par la formule suivante :

$$CA = E\left( A / (\frac{\sqrt{3} pn_0{}^2}{2}) \right) = E\left( \frac{2A}{\sqrt{3} pn_0{}^2} \right),$$

où E est la fonction Arrondie renvoyant l'entier le plus proche.

Une exécution de la boucle d'étapes 104, 106 est repérée par un indice $j \ge 1$. Au cours d'une j-ième exécution de l'étape 104, le simulateur 18 du module de traitement 12 fournit une nouvelle figure d'interférence $I_j$ produite à l'intérieur A du contour C par la simulation d'une propagation d'ondes interférant entre elles à partir des points de l'ensemble $E_{j-1}$ qui sont considérés comme des points d'émissions de ces ondes.

Un nouveau modèle de propagation fournissant de bons résultats pour la boucle d'étapes 104, 106 est indiqué à titre d'exemple ci-dessous. Ce modèle est défini de telle sorte qu'en tout point M situé à l'intérieur A du contour C, la nouvelle figure d'interférence $I_j$ prend la valeur suivante :

$$I_j(M) = I_0(M) + C_0 \sum_{E \in E_{j-1}} g\left(\left\|\overrightarrow{EM}\right\|\right) e^{\frac{2i\pi\left(\left\|\overrightarrow{EM}\right\| - 0{,}75\,pn_0\right)}{pn_0}} \quad,$$

où la fonction d'atténuation g est définie de la façon suivante :

$$g(r) = \begin{cases} 1 & si \quad r \leq pn_0 \\ e^{\frac{(r-pn_0)^2}{2d_2{}^2}} \cdot \left(1 - e^{\frac{9r^2}{2pn_0{}^2}}\right) & si \quad r > pn_0 \end{cases}.$$

Dans ces expressions, le paramètre $C_0$ est choisi par exemple proche de 1. Il peut aussi être défini à partir d'une calibration préalable du modèle, comme les paramètres $d_1$ et $d_2$.

Au cours de cette même étape 104, au moins un extremum local de la nouvelle figure d'interférence $I_j$ dans l'intérieur A du contour C, en l'occurrence un maximum local, est sélectionné. On note alors $E_j$ l'ensemble des extrema locaux de $I_j$. Le critère de sélection, et donc de constitution de l'ensemble $E_j$, est par exemple le suivant :

$$Ej = \left\{ M \in A \,\middle|\, \exists \varepsilon \in \Re^{+*} \,\middle|\, \forall M' \in A \;\; et \;\; 0 < \left\|\overrightarrow{MM'}\right\| < \varepsilon \Rightarrow \mathrm{Im}\left(I_j(M)\right) > \mathrm{Im}\left(I_j(M')\right) \right\}.$$

Au cours de l'étape suivante de test 106, le nombre de points dans l'ensemble $E_j$ est comparé à CA. Tant que le nombre CA n'est pas atteint, j est incrémenté d'une unité et le procédé reprend à l'étape 104 pour une nouvelle itération de la boucle d'étapes 104, 106.

Si CA est atteint, on passe à une étape finale 108 de fin de simulation, au cours de laquelle l'ensemble M de paramètres de placement de motifs dans le contour C est fourni. Plus exactement, M = $E_j$. Autrement dit, on considère que le placement des motifs destinés à être obtenus par auto assemblage de copolymères à blocs dans le contour C se fait sur les extrema locaux de la dernière figure d'interférence calculée.

La figure 4 illustre le résultat d'une exécution du procédé de simulation de la figure 2 sur un contour C simple, à l'intérieur duquel deux motifs peuvent être a priori placés par auto assemblage de copolymères à blocs. L'ensemble P est constitué de points $p_i$ répartis le long du contour C. L'ensemble M fourni en fin de simulation comporte deux motifs centrés en deux points $m_1$ et $m_2$.

Le procédé illustré sur la figure 5 est un procédé de conception d'un circuit imprimé destiné à comporter au moins un contour imprimé sur plaque par lithographie, au moins un motif devant être placé par auto assemblage de copolymères à blocs dans ce contour.

Il comporte une première étape 200 d'enregistrement en mémoire 14 des paramètres géométriques d'une pluralité de contours $C_1$, ..., $C_N$ de formes différentes. Cette première étape 200 est réalisée par exécution du premier programme d'ordinateur 16 et fournit la pluralité d'ensembles $P_1$, ..., $P_N$ de paramètres géométriques respectifs des contours $C_1$, ..., $C_N$.

Il comporte ensuite une étape 202 d'exécution des étapes 100 à 108 du procédé de simulation précédemment détaillé pour chacun des contours $C_1$, ..., $C_N$. Cette étape 202 est réalisée par exécution du deuxième programme d'ordinateur 18 et fournit la pluralité d'ensembles $M_i$, .... $M_N$ de paramètres de placement de motifs.

Il comporte ensuite une étape 204 de sélection d'un contour $C_S$ exécutée par le troisième programme d'ordinateur 20 dont le fonctionnement a déjà été détaillé.

Il comporte ensuite une étape 206 d'impression effective du contour sélectionné sur une plaque du circuit imprimé par photolithographie. Cette étape ne sera pas détaillée parce que bien connue.

Enfin, il comporte une étape 208 d'insertion et auto assemblage de copolymères à blocs dans le contour imprimé pour placer le ou les motifs souhaités. Cette étape ne sera pas détaillée non plus parce que bien connue également.

Il apparaît clairement qu'un système de conception, qu'un procédé de simulation et qu'un procédé de conception tels que ceux décrits précédemment permettent de faciliter la fabrication de circuits imprimés dans lesquels des motifs sont formés par auto assemblage de copolymères à blocs dans des contours imprimés sur plaque par lithographie. En proposant un modèle de placement des motifs original et simple à simuler, les performances de fabrication sont en effet améliorées.

On notera par ailleurs que l'invention n'est pas limitée aux modes de réalisation décrits précédemment.

En particulier, les paramètres $C_0$, $d_1$ et $d_2$ des modèles de propagation présentés peuvent prendre des valeurs différentes de celles choisies. Ils peuvent avantageusement être définis à partir d'un apprentissage sur quelques motifs. L'homme du métier saura les adapter au contexte. De même, les modèles de propagation peuvent être choisis différemment, en fonction du contexte également.

En particulier également, si une approche itérative est choisie pour estimer une figure d'interférence à l'intérieur d'un contour donné comme cela a été détaillé précédemment, le critère d'arrêt peut être différent de celui proposé. En variante, on peut par exemple déterminer à l'avance le nombre d'itérations. D'une façon générale, cinq itérations au plus suffisent. Par ailleurs, le critère d'arrêt pourrait être atteint, donc testé, dès l'exécution de l'étape 102, sans nécessiter alors d'exécution de la boucle d'étapes 104, 106.

Il apparaîtra plus généralement à l'homme de l'art que diverses modifications peuvent être apportées aux modes de réalisation décrits ci-dessus, à la lumière de l'enseignement qui vient de lui être divulgué. Dans les revendications qui suivent, les termes utilisés ne doivent pas être interprétés comme limitant les revendications aux modes de réalisation exposés dans la présente description, mais doivent être interprétés pour y inclure tous les équivalents que les revendications visent à couvrir du fait de leur formulation et dont la prévision est à la portée de l'homme de l'art en appliquant ses connaissances générales à la mise en oeuvre de l'enseignement qui vient de lui être divulgué.

**Revendications**

1. Procédé de simulation d'un placement de motifs ($m_1$, $m_2$) par auto assemblage de copolymères à blocs dans un contour (C) imprimé sur plaque par lithographie, comportant les étapes suivantes :

    - extraction (100) de paramètres géométriques (P) du contour (C) enregistrés en mémoire (14),
    - sélection (102, 104, 106), par un processeur (12) ayant accès à la mémoire (14), d'au moins un extremum local d'une figure d'interférence produite à l'intérieur du contour à partir des paramètres géométriques (P) du contour (C) par application d'un modèle de propagation d'ondes interférant entre elles,
    - fourniture (108), par le processeur (12) et à partir de cet extremum local, de paramètres (M) de placement d'au moins un motif destiné à être obtenu par auto assemblage de copolymères à blocs dans le contour (C).

2. Procédé de simulation selon la revendication 1, dans lequel :

    - les paramètres géométriques (P) du contour sont des points de ce contour,
    - l'étape de sélection (102, 104, 106) comporte l'application (102) d'un premier modèle de propagation d'ondes émises à partir de ces points, produisant une figure d'interférence à l'intérieur du contour (C), et la sélection (102) d'au moins un maximum local de la partie imaginaire de cette figure d'interférence.

3. Procédé de simulation selon la revendication 2, dans lequel la figure d'interférence produite par le premier modèle, notée $I_0(M)$ en un point M quelconque situé à l'intérieur du contour (C), est définie par l'expression suivante :

$$I_0\left(M\right) = \sum_{i=0}^{n-1} f\left(\left\|\overrightarrow{p_i M}\right\|\right) e^{\frac{2i\pi\left(\left\|\overrightarrow{p_i M}\right\| - d_1\right)}{p n_0}},$$

où f est une fonction d'atténuation définie de la façon suivante :

$$f(r) = \begin{cases} 1 & si & r \le d_1 \\ e^{-\frac{(r-d_1)^2}{2 d_2^2}} & si & r > d_1 \end{cases},$$

et où $\{p_i, 0 \le i < n\}$ est un ensemble de points du contour (C) formant ses paramètres géométriques (P), le paramètre

$d_1$ est un paramètre constant, par exemple choisi proche de la moitié d'un pas naturel desdits copolymères à blocs, ce pas naturel représentant une distance naturelle entre copolymères à blocs à l'équilibre énergétique, et le paramètre $d_2$ est un paramètre constant, par exemple choisi proche de ce pas naturel.

4. Procédé de simulation selon la revendication 3, dans lequel les paramètres $d_1$ et $d_2$ sont définis à partir d'une calibration dudit modèle de propagation sur quelques motifs.

5. Procédé de simulation selon l'une quelconque des revendications 2 à 4, dans lequel l'étape de sélection (102, 104, 106) comporte en outre, suite à l'application dudit premier modèle de propagation et à la sélection d'au moins un maximum local de la partie imaginaire de la figure d'interférence produite par ce premier modèle, une boucle d'étapes (104, 106) comportant l'application d'un nouveau modèle de propagation d'ondes, chaque itération de cette boucle d'étapes comportant :

- l'application (104) du nouveau modèle par émissions d'ondes à partir d'au moins un maximum local sélectionné à l'itération précédente ou à partir dudit au moins un maximum local sélectionné par application du premier modèle s'il s'agit de la première itération, produisant une nouvelle figure d'interférence à l'intérieur du contour (C),
- la sélection (104) d'au moins un maximum local de la partie imaginaire de cette nouvelle figure d'interférence.

6. Procédé de simulation selon la revendication 5, dans lequel la nouvelle figure d'interférence, notée $I_j(M)$ en un point M quelconque situé à l'intérieur du contour (C) et à la j-ème itération de la boucle d'étapes (104, 106), est définie par l'expression suivante :

$$I_j(M) = I_0(M) + C_0 \sum_{E \in E_{j-1}} g\left(\left\|\overrightarrow{EM}\right\|\right) e^{\frac{2i\pi\left(\left\|\overrightarrow{EM}\right\| - 0,75\, pn_0\right)}{pn_0}},$$

où la fonction d'atténuation g est définie de la façon suivante :

$$g(r) = \begin{cases} 1 & si \quad r \le pn_0 \\ e^{\frac{(r - pn_0)^2}{2d_2^2}} \cdot \left(1 - e^{-\frac{9r^2}{2pn_0^2}}\right) & si \quad r > pn_0 \end{cases},$$

et où $C_0$ est un paramètre constant, par exemple choisi proche de 1, et $E_{j-1}$ est l'ensemble des maxima locaux sélectionnés à l'itération précédente ou par application du premier modèle s'il s'agit de la première itération.

7. Procédé de simulation selon la revendication 6, dans lequel le paramètre $C_0$ est défini à partir d'une calibration dudit modèle de propagation sur quelques motifs.

8. Procédé de simulation selon l'une quelconque des revendications 1 à 7, dans lequel l'étape de sélection (102, 104, 106) s'arrête lorsque le nombre d'extrema locaux sélectionnés atteint un nombre attendu de motifs à placer dans le contour (C) par auto assemblage de copolymères à blocs, ce nombre attendu étant par exemple défini comme étant le ratio entre la surface intérieure du contour (C) et un espace naturel moyen connu, occupé par chacun de ces motifs dans un environnement sans contrainte de contour, pour leur placement par auto assemblage de copolymères à blocs.

9. Procédé de conception d'un circuit imprimé destiné à comporter au moins un contour (C) imprimé sur plaque par lithographie, au moins un motif devant être placé par auto assemblage de copolymères à blocs dans ce contour, comportant les étapes suivantes :

- enregistrement (200) en mémoire (14) de paramètres géométriques ($P_1$, ..., $P_N$) d'une pluralité de contours de formes différentes,
- exécution (202) des étapes (100, 102, 104, 106, 108) d'un procédé de simulation selon l'une quelconque des revendications 1 à 8 pour chacun desdits contours, et

- sélection (204) d'un contour ($C_S$) à partir des paramètres de placement ($M_1$, ..., $M_N$) fournis pour chacun desdits contours, sur la base d'un placement ($M_S$) souhaité prédéfini d'au moins un motif dans le circuit imprimé.

10. Procédé de conception d'un circuit imprimé selon la revendication 9, comportant en outre les étapes suivantes :

- impression (206) du contour sélectionné ($C_S$) sur une plaque du circuit imprimé par photolithographie, et
- insertion (208) et auto assemblage de copolymères à blocs dans ce contour imprimé.

11. Programme d'ordinateur (16, 18, 20) téléchargeable depuis un réseau de communication et/ou enregistré sur un support lisible par ordinateur et/ou exécutable par un processeur, **caractérisé en ce qu'**il comprend des instructions pour l'exécution des étapes d'un procédé de simulation selon l'une quelconque des revendications 1 à 8 ou pour l'exécution des étapes d'un procédé de conception selon la revendication 9 ou 10, lorsque ledit programme est exécuté sur un ordinateur.

12. Système (10) de conception de circuit imprimé, **caractérisé en ce qu'**il comporte :

- une mémoire (14) de stockage de paramètres géométriques ($P_1$, ..., $P_N$) d'une pluralité de contours de formes différentes,
- un simulateur (18) programmé pour la mise en oeuvre d'un procédé de simulation selon l'une quelconque des revendications 1 à 8 pour chacun desdits contours, et
- un sélecteur de contour (20) à partir des paramètres de placement ($M_1$, ..., $M_N$) fournis pour chacun desdits contours, sur la base d'un placement ($M_S$) souhaité prédéfini d'au moins un motif dans le circuit imprimé.

**Patentansprüche**

1. Verfahren zur Simulation einer Platzierung von Mustern ($m_1$, $m_2$) durch Selbstassemblierung von Copolymeren mit Blöcken in einer Kontur (C), die auf eine Platte durch Lithographie gedruckt wird, das die folgenden Schritte umfasst:

- Extraktion (100) geometrischer Parameter (P) der Kontur (C), die in einem Speicher (14) gespeichert sind,
- Auswahl (102, 104, 106) durch einen Prozessor (12), der Zugang zu dem Speicher (14) hat, mindestens eines lokalen Extremums eines Interferenzmusters, das im Inneren der Kontur erzeugt wird, ausgehend von den geometrischen Parametern (P) der Kontur (C) durch Anwenden eines Ausbreitungsmodells von Wellen, die miteinander interferieren,
- Bereitstellen (108) durch den Prozessor (12) und ausgehend von diesem lokalen Extremum von Platzierungsparametern (M), mindestens eines Musters, das dazu bestimmt ist, durch Selbstassemblierung von Copolymeren mit Blöcken in der Kontur (C) erhalten zu werden.

2. Simulationsverfahren nach Anspruch 1, bei dem:

- die geometrischen Parameter (P) der Kontur Punkte dieser Kontur sind,
- der Auswahlschritt (102, 104, 106) das Anwenden (102) eines ersten Ausbreitungsmodells von Wellen, die ausgehend von diesen Punkten gesendet werden, umfasst, die ein Interferenzmuster im Inneren der Kontur (C) erzeugen, und die Auswahl (102) mindestens eines lokalen Maximums des imaginären Teils dieses Interferenzmusters.

3. Simulationsverfahren nach Anspruch 2, wobei das Interferenzmuster, das durch das erste Modell erzeugt wird, $I_0(M)$ genannt, in einem beliebigen Punkt M, der in dem Inneren der Kontur (C) liegt, durch die folgende Gleichung definiert ist:

$$I_0\left(M\right) = \sum_{i=0}^{n-1} f\left(\left\|\overrightarrow{p_i M}\right\|\right) e^{\frac{2i\pi\left(\left\|\overrightarrow{p_i M}\right\| - d_1\right)}{p n_0}} \; ,$$

wobei f eine Dämpfungsfunktion ist, die wie folgt definiert ist:

$$f(r) = \begin{cases} 1 & wenn \quad r \le d_1 \\ e^{-\frac{(r-d_1)^2}{2d_2^2}} & wenn \quad r > d_1 \end{cases},$$

und wobei $\{p_i, 0 \le i < n\}$ eine Einheit von Punkten der Kontur (C) ist, die ihre geometrischen Parameter (P) bildet, der Parameter $d_1$ ein konstanter Parameter ist, der zum Beispiel nahe der Hälfte einer natürlichen Stufe der Copolymere mit Blöcken ausgewählt ist, wobei diese natürliche Stufe eine natürliche Distanz zwischen Copolymeren mit Blöcken zu dem energetischen Gleichgewicht darstellt, und der Parameter $d_2$ ein konstanter Parameter ist, der zum Beispiel nahe dieser natürlichen Stufe ausgewählt ist.

4. Simulationsverfahren nach Anspruch 3, wobei die Parameter $d_1$ und $d_2$ ausgehend von einer Kalibrierung des Ausbreitungsmodells auf einigen Mustern definiert sind.

5. Simulationsverfahren nach einem der Ansprüche 2 bis 4, wobei der Auswahlschritt (102, 104, 106) außerdem im Anschluss an das Anwenden des ersten Ausbreitungsmodells und an die Auswahl mindestens eines lokalen Maximums des imaginären Teils des Interferenzmusters, das durch dieses erste Modell erzeugt wird, eine Schleife von Schritten (104, 106) umfasst, die das Anwenden eines neuen Ausbreitungsmodells von Wellen umfasst, wobei jede Iteration dieser Schleife von Schritten Folgendes umfasst:

- das Anwenden (104) des neuen Modells durch Senden von Wellen ausgehend von mindestens einem lokalen Maximum, das bei der vorhergehenden Iteration ausgewählt wurde, oder ausgehend von dem mindestens einen lokalen Maximum, das durch Anwenden des ersten Modells ausgewählt wurde, wenn es sich um die erste Iteration handelt, das ein neues Interferenzmuster im Inneren der Kontur (C) erzeugt,
- die Auswahl (104) mindestens eines lokalen Maximums des imaginären Teils dieses neuen Interferenzmusters.

6. Simulationsverfahren nach Anspruch 5, wobei das neue Interferenzmuster, $I_j(M)$ genannt, in einem beliebigen Punkt M, der im Inneren der Kontur (C) liegt und bei der j. Iteration der Schleife von Schritten (104, 106) durch die folgende Gleichung definiert ist:

$$I_j(M) = I_0(M) + C_0 \sum_{E \in E_{j-1}} g\left(\left\|\overrightarrow{EM}\right\|\right) e^{\frac{2i\pi\left(\left\|\overrightarrow{EM}\right\| - 0,75\, pn_0\right)}{pn_0}},$$

wobei die Dämpfungsfunktion g wie folgt definiert ist:

$$g(r) = \begin{cases} 1 & wenn \quad r \le pn_0 \\ e^{-\frac{(r-pn_0)^2}{2d_2^2}} \cdot \left(1 - e^{-\frac{9r^2}{2pn_0^2}}\right) & wenn \quad r > pn_0 \end{cases},$$

und wobei $C_0$ ein konstanter Parameter ist, der zum Beispiel nahe an 1 ausgewählt ist, und $E_{j-1}$ die Einheit der lokalen Maxima ist, die bei der vorhergehenden Iteration oder durch Anwenden des ersten Modells, falls es sich um die erste Iteration handelt, ausgewählt wurden.

7. Simulationsverfahren nach Anspruch 6, wobei der Parameter $C_0$ ausgehend von einer Kalibrierung des Ausbreitungsmodells auf einigen Mustern definiert wird.

8. Simulationsverfahren nach einem der Ansprüche 1 bis 7, wobei der Auswahlschritt (102, 104, 106) stoppt, wenn die Anzahl lokaler Extrema eine erwartete Anzahl von Mustern erreicht, die in der Kontur (C) durch Selbstassemblierung von Copolymeren mit Blöcken zu platzieren ist, wobei diese erwartete Anzahl zum Beispiel als das Verhältnis zwischen der inneren Oberfläche der Kontur (C) und einem bekannten mittleren natürlichen Raum ist, der von jedem dieser Muster in einer Umgebung ohne Konturzwang belegt wird, für ihre Platzierung durch Selbstassemblierung von Copolymeren mit Blöcken.

9. Verfahren zur Konzeption einer Leiterplatte, die dazu bestimmt ist, mindestens eine Kontur (C) zu umfassen, die auf die Platte durch Lithographie gedruckt wird, wobei mindestens ein Muster durch Selbstassemblierung von Copolymeren mit Blöcken in dieser Kontur platziert werden soll, das die folgenden Schritte umfasst:

- Aufzeichnen (200) im Speicher (14) geometrischer Parameter ($P_1$, ..., $P_N$) einer Mehrzahl von Konturen mit unterschiedlichen Formen,
- Ausführen (202) der Schritte (100, 102, 104, 106, 108) eines Simulationsverfahrens nach einem der Ansprüche 1 bis 8 für jede der Konturen, und
- Auswahl (204) einer Kontur ($C_S$) ausgehend von den Platzierungsparametern ($M_1$, ..., $M_N$), die für jede der Konturen geliefert werden, auf der Basis einer vorbestimmten gewünschten Platzierung ($M_S$) mindestens eines Musters in der Leiterplatte.

10. Verfahren zur Konzeption einer Leiterplatte nach Anspruch 9, das außerdem die folgenden Schritte umfasst:

- Drucken (206) der ausgewählten Kontur ($C_S$) auf eine Platte der Leiterplatte durch Fotolithographie, und
- Einfügen (208) und Selbstassemblierung von Copolymeren mit Blöcken in dieser gedruckten Kontur.

11. Computerprogramm (16, 18, 20), das ausgehend von einem Kommunikationsnetzwerk heruntergeladen werden kann und/oder auf einem Träger aufgezeichnet ist, das von einem Computer lesbar und/oder von einem Prozessor ausführbar ist, **dadurch gekennzeichnet, dass** es Anweisungen zur Ausführung der Schritte eines Simulationsverfahrens nach einem der Ansprüche 1 bis 8 oder zur Ausführung der Schritte eines Konzeptionsverfahrens nach Anspruch 9 oder 10 umfasst, wobei das Programm auf einem Computer ausgeführt wird.

12. System (10) zur Konzeption einer Leiterplatte, **dadurch gekennzeichnet, dass** es Folgendes umfasst:

- einen Speicher (14) zum Speichern geometrischer Parameter ($P_1$, ..., $P_N$) einer Mehrzahl von Konturen mit unterschiedlichen Formen,
- einen Simulator (18), der für das Umsetzen eines Simulationsverfahrens nach einem der Ansprüche 1 bis 8 für jede der Konturen programmiert ist, und
- einen Konturauswähler (20) ausgehend von den Platzierungsparametern ($M_1$, ..., $M_N$), die für jede der Konturen geliefert werden, auf der Basis einer vordefinierten gewünschten Platzierung ($M_S$) mindestens eines Musters in der Leiterplatte.

**Claims**

1. A method for simulating a placement of patterns ($m_1$, $m_2$) by self-assembly of block copolymers within a contour (C) printed on a plate by lithography, including the following steps:

- extraction (100) of geometric parameters (P) of the contour (C) recorded in a memory (14),
- selection (102, 104, 106), by a processor (12) having access to the memory (14), of at least one local extremum of an interference figure produced inside the contour on the basis of the geometric parameters (P) of the contour (C) by applying a model for propagation of waves interfering with one another,
- provision (108), by the processor (12) and on the basis of this local extremum, of parameters (M) for placing at least one pattern intended to be obtained by self-assembly of block copolymers within the contour (C).

2. The simulation method as claimed in claim 1, wherein:

- the geometric parameters (P) of the contour are points of said contour,
- the selection step (102, 104, 106) includes the application (102) of a first model for propagation of waves transmitted from these points, producing an interference figure inside the contour (C), and the selection (102) of at least one local maximum of the imaginary part of said interference figure.

3. The simulation method as claimed in claim 2, wherein the interference figure produced by the first model, denoted $I_0(M)$ at any point M located inside the contour (C), is defined by the following expression:

$$I_0(M) = \sum_{i=0}^{n-1} f\left(\left\|\overrightarrow{p_iM}\right\|\right) e^{\frac{2i\pi\left(\left\|\overrightarrow{p_iM}\right\|-d_1\right)}{pn_0}},$$

where f is an attenuation function defined as follows:

$$f(r) = \begin{cases} 1 & if \quad r \le d_1 \\ e^{-\frac{(r-d_1)^2}{2d_2^2}} & if \quad r > d_1 \end{cases},$$

and where $\{p_i, 0 \le i < n\}$ is a set of points of the contour (C) forming its geometric parameters (P), parameter $d_1$ is a constant parameter, for example chosen to be close to half of a natural pitch of said block copolymers, said natural pitch representing a natural distance between block copolymers at energy equilibrium, and parameter $d_2$ is a constant parameter, for example chosen to be close to said natural pitch.

4. The simulation method as claimed in claim 3, wherein parameters $d_1$ and $d_2$ are defined on the basis of a calibration of said propagation model on several patterns.

5. The simulation method as claimed in any one of claims 2 to 4, wherein the selection step (102, 104, 106) further comprises, following the application of said first propagation model and the selection of at least one local maximum of the imaginary part of the interference figure produced by said first model, a loop of steps (104, 106) comprising the application of a new wave propagation model, each iteration of said loop of steps comprising:

- the application (104) of the new model by wave emissions from at least one local maximum selected in the previous iteration or from said at least one local maximum selected by applying the first model if it is the first iteration, producing a new interference figure inside the contour (C),
- the selection (104) of at least one local maximum of the imaginary part of said new interference figure.

6. The simulation method as claimed in claim 5, wherein the new interference figure, denoted $I_j(M)$ at any one point M located inside the contour (C) and at the j-th iteration of the loop of steps (104, 106), is defined by the following expression:

$$I_j(M) = I_0(M) + C_0 \sum_{E \in E_{j-1}} g\left(\left\|\overrightarrow{EM}\right\|\right) e^{\frac{2i\pi\left(\left\|\overrightarrow{EM}\right\|-0.75pn_0\right)}{pn_0}},$$

where the attenuation function g is defined as follows:

$$g(r) = \begin{cases} 1 & if \quad r \le pn_0 \\ e^{-\frac{(r-pn_0)^2}{2d_2^2}} \cdot \left(1 - e^{-\frac{9r^2}{2pn_0^2}}\right) & if \quad r > pn_0 \end{cases},$$

and where $C_0$ is a constant parameter, for example chosen to be close to 1, and $E_{j-1}$ is the set of local maxima selected in the previous iteration or by applying the first model if it is the first iteration.

7. The simulation method as claimed in claim 6, wherein the parameter $C_0$ is defined on the basis of a calibration of said propagation model on several patterns.

8. The simulation method as claimed in any one of claims 1 to 7, wherein the selection step (102, 104, 106) is stopped once the number of local extrema selected reaches an expected number of patterns to be placed within the contour (C) by self-assembly of block copolymers, said expected number being, for example, defined as being the ratio between the interior surface of the contour (C) and a known natural mean space, occupied by each of said patterns in an environment without contour constraints, for their placement by self-assembly of block copolymers.

9. A method for designing a printed circuit intended to include at least one contour (C) printed on a plate by lithography, at least one pattern having to be placed by self-assembly of block copolymers within said contour, including the following steps:

- recording (200) in a memory (14) of geometric parameters ($P_1$, ..., $P_N$) of a plurality of contours of different shapes,
- execution (202) of steps (100, 102, 104, 106, 108) of a simulation method as claimed in any one of claims 1 to 8 for each of said contours, and
- selection (204) of a contour ($C_S$) on the basis of the placement parameters ($M_1$, ..., $M_N$) provided for each of said contours, on the basis of a predefined desired placement ($M_S$) of at least one pattern in the printed circuit.

10. The method for designing a printed circuit as claimed in claim 9, further including the following steps:

- printing (206) of the selected contour ($C_S$) on a plate of the printed circuit by photolithography, and
- insertion (208) and self-assembly of block copolymers within said printed contour.

11. A computer program (16, 18, 20) downloadable from a communication network and/or recorded on a computer readable medium and/or executable by a processor, **characterized in that** it includes instructions for executing steps of a simulation method as claimed in any one of claims 1 to 8 or for executing steps of a design method as claimed in claim 9 or 10, when said program is executed on a computer.

12. A system (10) for designing a printed circuit, **characterized in that** it includes:

- a memory (14) for storing geometric parameters ($P_1$, ..., $P_N$) of a plurality of contours of different shapes,
- a simulator (18) programmed for implementing a simulation method as claimed in any one of claims 1 to 8 for each of said contours, and
- a contour selector (20) for selecting a contour on the basis of the placement parameters ($M_1$, ..., $M_N$) provided for each of said contours, on the basis of a predefined desired placement ($M_S$) of at least one pattern in the printed circuit.

## *Figure 1*

## *Figure 2*

## Figure 3

$$pn_0$$

Esp

## Figure 4

$p_i$

$C$

$m_1$

$m_2$

## Figure 5

200

202

204

206

208

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **SANDERS et al.** Integration of directed self-assembly with 193 nm lithography. *Journal of Photopolymer Science and Technology,* 2010, vol. 23 (1), 11-18